# EUROPEAN PATENT APPLICATION

(11) **EP 3 505 996 A1**
(43) Date of publication of application: **03.07.2019**
(21) Application number: 18198060.8
(22) Date of filing: 01.10.2018
(51) Int. Cl.: G02C 7/04

(54) **DUAL DEFOCUS LENS**

(30) Priority: 27.12.2017 TW 106145914
(71) Applicant: UNICON Optical Co, LTD., Baoshan Township, Hsinchu County 30075 (TW)
(72) Inventor: Hsiao, Hsu-Kuei, 408 Taichung City (TW); Chen, Chih-Cheng, 406 Taichung City (TW); Liao, Hsien-Sheng, 40760 Taichung City (TW)
(74) Representative: Lang, Christian

(57) **Abstract**

A dual defocus lens is disclosed in the present invention. The dual defocus lens comprises a refractive-error correction part, a positioning part and a periphery part. The refractive-error correction part comprises a central optical region, a first defocus region and a second defocus region. The first defocus region, the second defocus region, the positioning part and the periphery part are all concentric to the central optical region having the same center as the refractive-error correction part. The first defocus region and the central optical region have different diopters individually for correcting the positions of imaging on the retina. The second defocus region having small radius of curvature provides shorter focus for imaging in the front of the retina. Thus, the retina will not be elongated or deformed gradually due to the image beyond the retina so that the myopia can be greatly controlled.

## Description

This application claims priority for Taiwan patent application no. 106145914 filed on December 27, 2017, the content of which is incorporated by reference in its entirety.

### BACKGROUND OF THE INVENTION

### Technical Field

The present invention is related to a lens, particularly related to a dual defocus lens.

### Description of Related Art

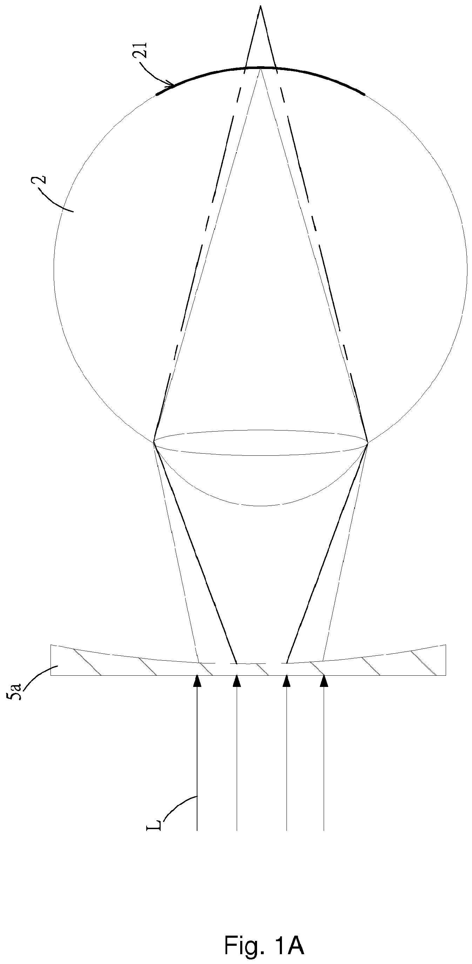

In order to solve the poor vision more efficiently and correctly, the design of the lens is focused on the correcting the refractive error instead of on improving the visual resolution. Take the myopia as an example, the images of different incident angles should be imaged on different positions of the retina 21 having a curved surface. The retinal image is quite poor owing to the only one diopter provided via the lens 5a. No different incident angles of lights can be generated via the lens 5a. The light L through the lens 5a is split and only some part of the split light corresponding to the central region of the retina 21 can form a clear retinal image while other part of the split light is focused behind the retina 21 (illustrated as the dashed line) cannot form the retinal image at all. As illustrated in FIG. 1A, the retina 21 will deform backwards after long-time usage of the lens 5a. Consequently, the diopter cannot be improved, and the vision gets worse either.

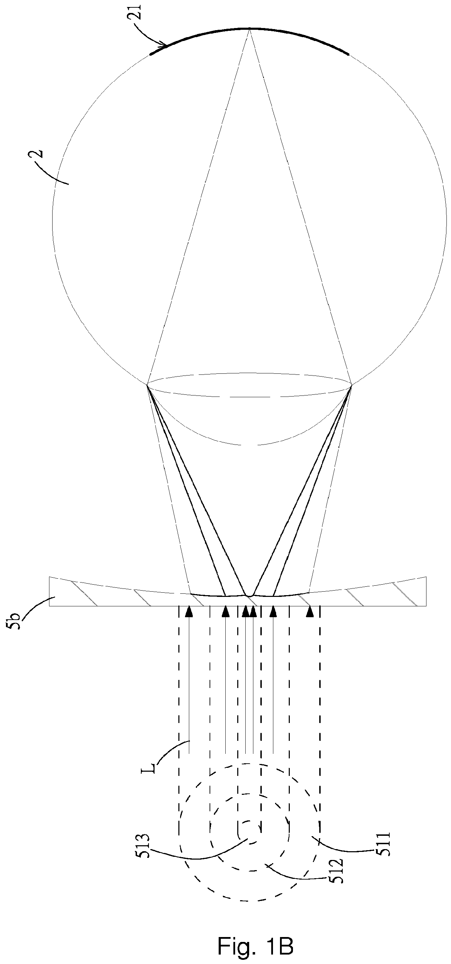

Later, a multifocal lens is developed and is illustrated in FIG. 1B. Generally, the multifocal lens 5b comprises many concentric-ring optical regions, as illustrated as the dashed line. The inner central region 513 is surrounded via the outer concentric-ring regions 511 and 512, as illustrated in FIG. 1B. The inner central region 513 has a first focal length, the outer concentric-ring region 511 has a second focal length and the concentric-ring region 512 has a third focal length, wherein the first, second and the third focal lengths are different. Hence, the light L passing through the inner central region 513 images on the corresponding region of the retina 21; the light L passing through the outer concentric-ring regions 511 and 512 image on the corresponding region of the retina 21 as well. The multifocal lens 5b can be suitable for both myopia vision and hyperopia vision, however, the rapid and frequent of relaxation and contraction of the ciliary muscle lead to instant vertigo or short-sighted blindness during the transition of myopia vision to hyperopia vision or hyperopia vision to myopia vision. Besides, most of the multifocal lenses are mainly non-contact type. The reason is that the optical regions having different focuses can be more easily processed because the non-contact hard lenses have rigid material properties and larger area. Comparing to the soft lenses having soft material properties and smaller area, the focal lengths for each of optical regions can be precisely controlled under the lower cost situation for the non-contact hard lenses.

Accordingly, the dual defocus lens disclosed in the present invention can correct vision not only via reversion regulation of the diopter for slowing down the increasement of the diopter but also via imaging the retinal images in front of the retina. Thus, the retina would not deform backwards after long-time usage of the dual defocus lens disclosed in the present invention.

### BRIEF SUMMARY OF THE INVENTION

The main purpose of the present invention is to provide a dual defocus lens including a central optical region and two defocus regions having different radii of curvatures. The retinal image of the central optical region is imaged within the central area of the retina. The image of the first defocus region is concentric to the retinal image of the central optical region; the image of the second defocus region is imaged in the front of the retina. Thus, the images of the defocus regions will not be imaged behind the retina and the diopter can be controlled via avoiding the deformation of the retina after a long-time usage of the dual defocus lens of the present invention.

Another purpose of the present invention is to provide a lens made of hydrogel or silicone hydrogel, which has a refractive-error correction part with various focuses. The refractive-error correction part includes the central optical region, the first defocus region, closely adjacent to the central optical region, and the second defocus region, positioned within the first defocus region. The corrections of the diopter for each of the regions are different. Comparing to the conventional hard lens, the lens of the present invention fits to the eyeball so that the correction of the diopter and the accuracy of the defocus are performed better. Meanwhile, the comfort and the effect of the diopter correction can be greatly improved.

Another purpose of the present invention is to provide a diopter correction from +0.25D to +8.0D progressively via the first defocus region. Hence, the correction of the diopter can be design within a range according to the demands. Unlike the conventional multifocal lens, in the present invention, the second defocus region is positioned within the first defocus region to ensure that the image can be imaged in front of the retina. The instant vertigo or short-sighted blindness caused via the rapid and frequent of relaxation and contraction of the ciliary muscle during the vision transition can be avoided.

Another purpose of the present invention is not only to provide better diopter correction but to reduce the production cost via improving the yield ratio for the design of the second defocus region as well.

For achieving the purposes mentioned above, a dual defocus lens is disclosed in the present invention. The dual defocus lens includes a refractive-error correction part, a positioning part and a periphery part. The refractive-error correction part comprises a central optical region, a first defocus region and a second defocus region, wherein the second defocus region is positioned within the area of the first defocus region and is closer to the positioning part. The first defocus region is positioned closely adjacent to the central optical region. The first defocus region, the second defocus region, the positioning part and the periphery part are all concentric to the central optical region having the same center as the refractive-error correction part. The dual defocus lens has various diopters so that the image of defocus region is imaged in front of the retina. Thus, the retina will not be elongated or deformed gradually due to the image beyond the retina and the myopia can be greatly controlled.

### BRIEF DESCRIPTION OF THE SEVERAL VIEWS OF THE DRAWINGS

FIG. 1A illustrates a cross-section view of a conventional single-focal hard lens.
FIG. 1B illustrates a cross-section view of a conventional multifocal hard lens.
FIG. 2 illustrates a top view and its corresponding cross-section view of a dual defocus lens of the present invention.
FIG. 3 illustrates a schematic diagram of retinal image imaged via a dual defocus lens of the present invention.

### DETAILED DESCRIPTION OF THE INVENTION

The embodiments of the present invention will be further illustrated by the following associated drawings. The skilled in the art can make various changes and modifications based on the contents of the present invention.

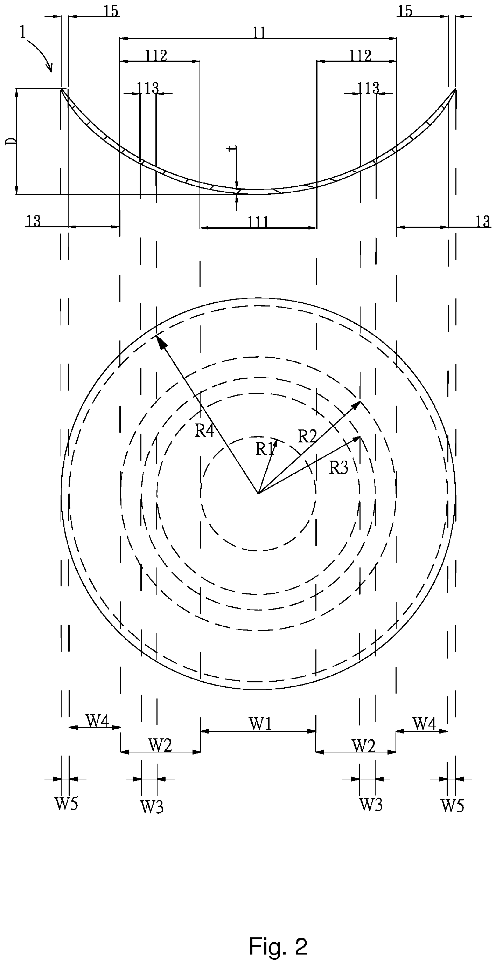

Refer to the FIG. 2, it illustrates a top view and its corresponding cross-section view of a dual defocus lens of the present invention. The dual defocus lens 1 includes a refractive-error correction part 11, a positioning part 13 and a periphery part 15. The refractive-error correction part 11comprises a central optical region 111, a first defocus region 112 and a second defocus region 113. The center of the central optical region 111 is positioned in the center of the refractive-error correction part 11. The central optical region 111, the first defocus region 112 and the second defocus region 113 are concentrically positioned. That is, the regions 111, 112 and 113 of the refractive-error correction part 11 are concentric. Also, the positioning part 13 and the periphery part 15 are concentric to the central optical region 111 of the refractive-error correction part 11. The first defocus region 112 is closely adjacent to the central optical region 111. The second defocus region 113 is positioned entirely within the first defocus region 112 and has no contact with both the central optical region 111 and the positioning part 13. The positioning part 13 is closely adjacent to the first defocus region 112; the outer edge of the positioning part 13 is directly contacted with the periphery part 15.

Besides, based on the curvature of the dual defocus lens 1, the first defocus region 112 and the central optical region 111 are concentric. The second defocus region 113, the positioning part 13 and the periphery part 15 can be concentric or non-concentric. The reference center is the center of the refractive-error correction part 11.

In order to achieve the purposes and to take the comfort as consideration, the material of the dual defocus lens 1 can be selected from hydrogel or silicone hydrogel. The specifications of the dual defocus lens 1 are illustrated as follow. The width W1 of the central optical region 111 the refractive-error correction part 11 is ranging from 3.00 mm to 6.00 mm. The width W2 of the first defocus region 112 is ranging from 1.00 mm to 3.00 mm. The width W3 of the second defocus region 113 is ranging from 0.10 mm to 0.15 mm. The width W4 of the positioning part 13 is ranging from 3.00 mm to 5.00 mm. The width W5 of the periphery part 15 is approximately 0.50 mm.

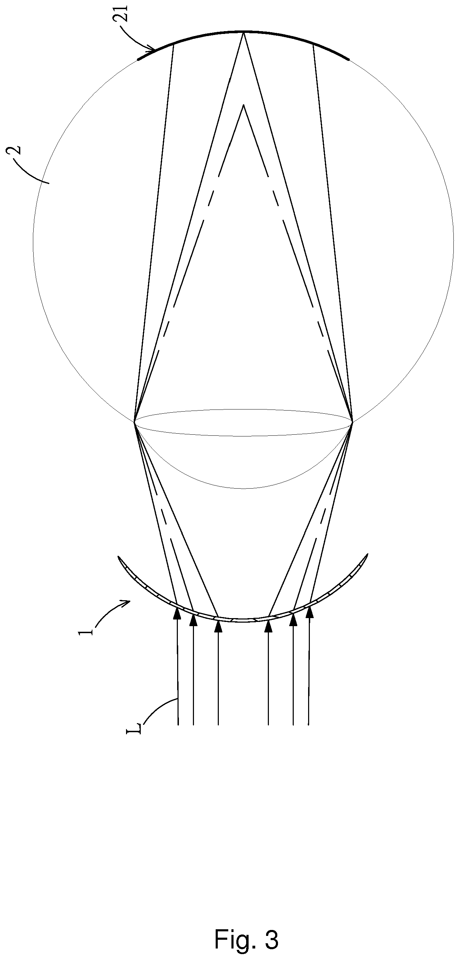

Further, refer to FIG. 2 and FIG. 3 illustrates a schematic diagram of retinal image imaged via a dual defocus lens of the present invention. The refractive-error correction part 11 is mainly the area for correction of the diopter. The light L passing through the eyeball 2 passes through the central optical region 111 and images a retinal image on central area of the retina 21. The image imaged via the first defocus region 112 is concentric to the retinal image imaged via the central optical region 111. The image imaged via the first defocus region 112 is positioned on the retina 21. Depending on characteristic of eyeball 2 and its related diopter, the position of the image imaged via the first defocus region 112 may be slightly varied. Generally, the correction of diopter of the first defocus region 112 is ranging from +0.25D to +8.0D. The image imaged via the second defocus region 113 is positioned behind the retina 21 instead of on the or in front of the retina 21 due to the curvature of the second defocus region 113. Hence, the retina 21 will not be elongated or deformed after a long-time usage. In order to achieve the purposes mentioned above, within the refractive-error correction part 11, the radius of curvature R1 of the central optical region 111 is larger than the radius of curvature R2 of the first defocus region 112; the radius of curvature R2 of the first defocus region 112 is larger than the radius of curvature R3 of the second defocus region 113. As for the parts 11 and 13, the radius of curvature R4 of the positioning part 13 is larger than the radius of curvature R1 of the central optical region 111. For the refractive-error correction part 11, the positions of the images imaged via the central optical region 111, the first defocus region 112 and the second defocus region 113 are determined via the radii of curvatures R1, R2 and R3. Comparing to the radii of curvatures R1 and R2 of the central optical region 111 and the first defocus region 112 respectively, the radius of curvature R3 of the second defocus region 113 is apparently smaller than the radii of curvatures R1 and R2 so that the image imaged via the second defocus region 113 is positioned in front of the retina 21. Basically, the ratio (R3/R1) of the radius of curvature R3 of the second defocus region 113to the radius of curvature R1 of the central optical region 111 is ranging from 0.30 to 0.80.

The shape of eyeball 2, such as corneal curvature, pupil size, shape of retina and so on, the focal length and so on are all the important factors for the width design and curvature design of the dual defocus lens 1. For instance, the radius of curvature R1 of the central optical region 111 of the refractive-error correction part 11 is larger than 8.00 mm, the radius of curvature R2 of the first defocus region 112 is less than 8.00 mm, and the radius of curvature R3 of the second defocus region 113 is ranging from 3.00 mm to 8.00 mm. The distance between the second defocus region 113 and an outer edge of the central optical region 111 is ranging from 1.00 mm to 5.00 mm, and the distance between the second defocus region 113 and an outer edge of the first defocus region 112 is less than 1.50 mm. Although the second defocus region 113 is concentric to the first defocus region 112 and the central optical region 111, the second defocus region 113 is positioned closer to the outer edge of the first defocus region 112, that is, the second defocus region 113 is closer to the positioning part 13 and away from the central optical region 111. It is noted that the image is mainly determined via the position of the second defocus region 113. For instance, too much light L would be pre-focused far away from the retina 21 and the image would be blurry or out-of-focus if the second defocus region 113 is positioned too close to the central optical region 111. Too much light L would pass through the first defocus region 112 and the image would be imaged only via a single diopter if the second defocus region 113 is positioned too far away from the central optical region 111 (too closer to the positioning part 13). Under this circumstance, the effect of the correction of diopter becomes poor. In order to solve this problem, the multiple defocus lens is required. However, the processes of the multiple defocus lens are complicated, low yield ratio and high production cost. Thus, the position of the second defocus region 113 must be positioned away from the central optical region 111 and be closer to the positioning part 13 surrounding the first defocus region 112.

Refer to FIG. 2, the thicknesses of the main regions of the dual defocus lens 1 are almost identical except the outer region of the periphery part 15. The thickness t is ranging from 0.10 mm to 0.20 mm. The distance D from the bottom to the top of the dual defocus lens 1 is approximately 3.50 mm±10%.

Furthermore, some more details are designed for the dual defocus lens of the present invention. Such that the slope of the central optical region and the first defocus region is smaller than a slope of the first defocus region and the positioning part. The reason for this design is that the central optical region and the first defocus region of the refractive-error correction part are mainly exerted for correction of diopter so the angle between the central optical region and the first defocus region is almost limited for fitting the eyeball smoothly and completely. In fact, a tear meniscus is exited between the refractive-error correction part and the cornea. The tear meniscus, a space filled with tears, has the function of defocus as well. However, in order to fit the lens to the eyeball smoothly and completely, the positioning part of the dual defocus lens of the present invention fits to the eyeball so that the larger slope exits between the first defocus region and the positioning part.

Accordingly, the dual defocus lens disclosed in the present invention provides the correction of the diopter not only via the central optical region of the refractive-error correction part but also via the first defocus region closely adjacent to the central optical region for imaging images with different focuses. Meanwhile, the second defocus region positioned within the first defocus region can shorten the focus of image so that some part of the light can be shaded. The light for imaging on the retina of the eyeball will not reach to behind the retina and the retina will not be elongated or deformed. Hence, the vision deterioration can be efficiently controlled.

The embodiments described above are intended only to demonstrate the technical concept and features of the present invention so as to enable a person skilled in the art to understand and implement the contents disclosed herein. It is understood that the disclosed embodiments are not to limit the scope of the present invention. Therefore, all equivalent changes or modifications based on the concept of the present invention should be encompassed by the appended claims.

## Claims

1. A dual defocus lens applied directly to an eyeball having a retina, comprising:
a refractive-error correction part, comprising:
a central optical region, been concentric to the refractive-error correction part, and having a retinal image of the central optical region imaged within a central area of the retina;
a first defocus region; been concentric to and closely adjacent to the central optical region, and having a retinal image of the first defocus region, which is concentrically to the retinal image of the central optical region, imaged within the central area of the retina; and
a second defocus region, positioned within the first defocus region and been concentric to and away from the central optical region, and having a retinal image of the second defocus region out of the retina, a radius of curvature of the first defocus region is larger than a radius of curvature of the second defocus region;
a positioning part, been concentric to the central optical region and been closely adjacent to the refractive-error correction part, a radius of curvature of the positioning part is larger than a radius of curvature of the central optical region which is larger than the radius of curvature of the first defocus region; and
a periphery part, been concentric to the central optical region and been closely adjacent to the positioning part.

2. The dual defocus lens of claim 1, wherein the retinal image of the second defocus region is imaged in front of the retina.

3. The dual defocus lens of claim 1, wherein the radius of curvature of the first defocus region is less than 8.00 mm, and the radius of curvature of the second defocus region is ranging from 3.00 mm to 8.00 mm.

4. The dual defocus lens of claim 3, wherein the radius of curvature of the central optical region is larger than 8.00 mm, and a ratio of the radius of curvature of the second defocus region to the radius of curvature of the central optical region is ranging from 0.30 to 0.80.

5. The dual defocus lens of claim 1, wherein a width of the central optical region is ranging from 3.00 mm to 6.00 mm, a width of the first defocus region is ranging from 1.00 mm to 3.00 mm, a width of the second defocus region is ranging from 0.10 mm to 0.15 mm, a width of the positioning part is ranging from 1.75 mm to 2.00 mm, and a width of the periphery part is approximately 0.50 mm.

6. The dual defocus lens of claim 1, wherein a distance between the second defocus region and an outer edge of the central optical region is ranging from 1.00 mm to 5.00 mm, and a distance between the second defocus region and an outer edge of the first defocus region is less than 1.50 mm.

7. The dual defocus lens of claim 1, wherein the second defocus region is closer to the positioning part than to the central optical region.

8. The dual defocus lens of claim 1, wherein the first defocus region provides a refractive power in diopter ranging from +0.25 D to +8.00 D.

9. The dual defocus lens of claim 1, wherein a slope of the central optical region and the first defocus region is smaller than a slope of the first defocus region and the positioning part.

10. The dual defocus lens of claim 1, which is made via hydrogel or silicone hydrogel.
